# EUROPÄISCHE PATENTSCHRIFT

(11) **EP 1 462 715 B1**
(45) Veröffentlichungstag und Bekanntmachung des Hinweises auf die Patenterteilung: **24.01.2007**
(21) Anmeldenummer: 04005686.3
(22) Anmeldetag: 10.03.2004
(51) Int. Cl.: F22B 1/28, A61H 33/12

(54) **Gerät zur Kräuterbedampfung**
Device for vaporising herbal extract
Dispositif pour la vaporisation des extraits d'herbes

(30) Priorität: 26.03.2003 DE 10313666; 08.07.2003 DE 10330681
(43) Veröffentlichungstag der Anmeldung: 29.09.2004
(73) Patentinhaber: Ionto-Comed GmbH, 76344 Eggenstein (DE)
(72) Erfinder: Senge, Wolfgang, 06792 Sandersdorf (DE)
(74) Vertreter: Brommer, Hans Joachim

(56) Entgegenhaltungen:
- WO-A-20/04018936
- DE-A- 4 109 357
- FR-A- 1 500 935
- US-A- 5 423 485
- US-B1- 6 236 808
- US-B1- 6 282 369

## Beschreibung

Die Erfindung betrifft ein Gerät zur Kräuterbedampfung für kosmetische Anwendungen. Ein solches Gerät dient zur Erzeugung von Wasserdampf, der mit ätherischen Ölen von Kräutern oder Pflanzenextrakten angereichert ist.

Dokument US-B1-6 282 369 offenbart ein solches Gerät zur Behandlung des Gesichts mit Dampf, wobei zwei Dampfabgabemöglichkeiten vorgesehen sind.

Die Kräuterbedampfung, die auch als Aromabedampfung bezeichnet wird, hat eine sehr positive Wirkung auf die Haut. Sie wirkt einerseits stoffwechselanregend, andererseits entspannend und beruhigend. Die Haut wird weicher und elastischer; Durchblutung und Stoffwechsel werden angeregt. Dadurch wird die Aufnahmefähigkeit der Haut für die spezielle Wirkung des entsprechenden Pflanzenextrakts bzw. ätherischen Öls gesteigert.

Das auf dem Markt befindliche Kräuterbedampfungsgerät "Ionto-Herb" der Ionto-Comed GmbH hat einen Dampftopf aus Edelstahl mit integrierter Heizplatte. Zum Befüllen des Dampftopfes dient ein abnehmbarer Deckel. Es wird immer die gesamte Wassermenge erhitzt. Im Dampfraum ist ein Kräutersieb angeordnet. Der mit ätherischen Ölen angereicherte Dampf wird über ein Dampfrohr an einen Dampfausgang geleitet. Durch die direkte Anordnung des Kräutersiebs über dem Dampftopf ist es unvermeidlich, dass mit Öl angereichertes Kondenswasser in den Topf zurückfließt. Der dadurch entstehende Schaum kann bis zu einem gewissen Maß akzeptiert werden. Erreicht der Schaum allerdings eine bestimmte Höhe, so muss die Heizung sofort abgeschaltet werden, um das Überschäumen des Bedampfers zu verhindern. Anderenfalls besteht die Gefahr, dass Schaum durch das Dampfrohr bis an den Dampfausgang gelangt, wodurch heiße Wassertröpfchen aus den zerplatzenden Schaumblasen zu Verbrühungen der zu behandelnden Person führen könnten. In diesem Zusammenhang spricht man vom gefürchteten "Spucken" des Bedampfers.

Das weiter entwickelte Kräuterbedampfungsgerät "Ionto-Herb XL" der Ionto-Comed GmbH hat einen Dampftopf, der über einen Einfüllstutzen mit abschraubbarem Verschluss bis zum maximalen Stand mit Wasser befüllt wird. Die elektrische Heizung bringt das Wasser im Dampftopf in einem Zuge zum Sieden. Der entstehende Dampf gelangt durch einen horizontalen Dampfkanal in einen Kräuterstutzen, wo eine Anreicherung mit ätherischen Ölen erfolgt. Der angereicherte Dampf gelangt dann weiter über die Austrittsöffnung eines Dampfrohrs ins Freie. Das im Dampfweg anfallende Kondensat läuft über einen Kondensatablauf in einen vom Dampftopf getrennten Kondensatbecher und wird dort aufgefangen. Das Kondensat und die darin enthaltenen ätherischen Öle gelangen somit nicht in den Dampftopf. Bei richtiger Betriebsweise kann somit kein Schaum entstehen, so dass in der Regel auch keine Gefahr eines Überschäumens oder gar "Spuckens" besteht. Allerdings muss der Kondensatbecher nach spätestens vier Bedampfungsvorgängen entleert werden, um ein Überfüllen zu verhindern. Zur Sicherheit wird die elektrische Heizung des Bedampfers bei maximalem Kondensatstand automatisch abgeschaltet. Nachteilig bei diesem Gerät ist ferner die ausladende Bauweise, die ein entsprechend stabiles Stativ mit schwerer Grundplatte erfordert.

Beiden vorbeschriebenen Kräuterbedampfungsgeräten gemeinsam ist die lange Aufheizzeit, da nach dem Befüllen stets die gesamte Wassermenge auf Siedetemperatur erhitzt werden muss. Durch eine aufwendige Regelelektronik mit Füllstandssensoren muss sichergestellt werden, dass die Heizung nur bei einwandfreien Betriebszuständen eingeschaltet ist.

Die Aufgabe besteht somit in der Schaffung eines verbesserten Gerätes zur Kräuterbedampfung, das sich bei konstruktiv einfachem und Platz sparendem Aufbau durch kurze Aufheizzeit, lange ununterbrochene Behandlungsdauer, bequeme Handhabung und hohe Betriebssicherheit auszeichnet.

Gelöst wird diese Aufgabe durch ein Gerät mit den Merkmalen des Anspruchs 1. Dieses Gerät zeichnet sich erfindungsgemäß durch eine Siedekammer, eine Sammelkammer und ein davon getrenntes Vorratsgefäß für kaltes Frischwasser aus. Das hat den großen Vorteil, dass die Siedekammer relativ klein ausgebildet werden kann, so dass nur eine geringe Wassermenge auf Siedetemperatur erhitzt werden muss. Dadurch verkürzt sich die Aufheizzeit nach dem Einschalten des Geräts sehr. Gleichzeitig wird aber auch die mögliche Dauer des ununterbrochenen Betriebs erheblich verlängert; sie ist letztlich nur durch das Fassungsvermögen des Vorratsgefäßes begrenzt. Durch das kleine Wasservolumen in der Siedekammer und die Möglichkeit, fortwährend frisches Wasser aus dem Vorratsgefäß zuführen zu können, ist die Neigung zur Schaumbildung von vornherein gering. Ein Überschäumen des Geräts, also ein Aufsteigen von Schaum durch das Dampfrohr bis in die Nähe der Austrittsöffnung, ist praktisch ausgeschlossen.

In vorteilhafter Weiterbildung des erfindungsgemäßen Kräuterbedampfungsgeräts ist das Vorratsgefäß für kaltes Wasser unterhalb der Siedekammer und der mit dieser kommunizierenden Sammelkammer angeordnet. Das kalte Wasser wird zweckmäßig durch eine elektrisch angetriebene Pumpe in der Zulaufleitung aus dem Vorratsgefäß in die Siedekammer gepumpt. Durch einen in der Sammelkammer angeordneten Überlauf, der in die Rücklaufleitung mündet, kann überschüssiges und/oder im Dampfraum gebildetes Kondensat zurück in das Vorratsgefäß fließen. Dabei bleibt der Wasserstand in der Siedekammer automatisch konstant, obwohl fortlaufend kaltes Frischwasser in die Siedekammer gelangt. Eine Anreicherung von ätherischen Ölen in der Siedekammer wird dadurch wirksam verhindert, so dass die Gefahr von Schaumbildung weiter herabgesetzt wird.

Vorteilhaft endet die Rücklaufleitung im Bereich des Bodens des Vorratsgefäßes. Bereits bei niedrigstem Füllstand im Vorratsgefäß endet die Rücklaufleitung damit unterhalb der Wasserlinie. Da das andere Ende der Rücklaufleitung über den Überlauf mit dem Dampfraum in Verbindung steht, stellt sich ein Druckgleichgewicht zwischen dem Dampfdruck im Dampfraum und dem aus Luftdruck und Wassersäule zusammengesetzten Gegendruck im unteren Abschnitt der Rücklaufleitung ein. Dieses Druckgleichgewicht funktioniert als Überdruckventil. Sollte der Dampfweg oder gar die Austrittsöffnung des Dampfrohres einmal verstopft sein, so kann der sich dann im Dampfraum ausbildende Überdruck über die Rücklaufleitung in das großvolumige Vorratsgefäß entweichen. Die Füllstandshöhe des im Vorratsgefäß stehenden Wassers begrenzt dabei den maximalen Druck im Dampfraum.

Besonders bevorzugt wird eine Ausführungsform, bei der die Siedekammer nach oben hin abgeschlossen ist und an der Seite Öffnungen für den Dampfaustritt ausweist, wobei die Sammelkammer an die Siedekammer angrenzt und der Dampfraum oberhalb der Siedekammer und der Sammelkammer ausgebildet ist. Im Dampfraum entstehendes Kondensat wird von der Siedekammer abgehalten und tropft automatisch in die daneben liegende Sammelkammer, aus der es zusammen mit dem überschüssigen Wasser in das Vorratsgefäß abgeleitet wird. Eine Aufkonzentrierung von ätherische Öle und/oder Pflanzenextrakt enthaltendes Kondensat in der Siedekammer wird dadurch wirksam verhindert. Gleichzeitig wird aber der Dampfaustritt durch die seitlich an der Siedekammer angebrachten Öffnungen nicht behindert.

Bevorzugt ist die Siedekammer zylindrisch ausgebildet, und befinden sich die Öffnungen für den Dampfaustritt in einem schmalen Band im oberen Abschnitt der Seitenwand. Die dann ebenfalls runde Sammelkammer ist zweckmäßig so ausgebildet, dass sie zumindest den oberen Teil der Dampfkammer konzentrisch umgibt. Das ergibt eine schmale Bauform des Geräts.

Eine weitere, vorteilhafte und durchaus - selbständig erfinderische Ausbildung des Kräuterbedampfungsgeräts sieht vor, dass die Zulaufleitung durch den Dampfraum und an den Öffnungen der Siedekammer vorbeigeführt ist. Ist die Siedekammer zylindrisch ausgebildet, verläuft die Zulaufleitung in einem Bogen um die Siedekammer herum. Damit sind zwei wichtige Vorteile verbunden: Zum einen wird das aus dem Vorratsgefäß hochgepumpte Wasser vorerwärmt, so dass der Temperaturunterschied beim Einfließen in die Siedekammer verringert wird, was Heizenergie spart und zu erhöhter thermischer Stabilität führt. Zum anderen treffen etwa durch die seitlichen Öffnungen der Siedekammer dringende Schaumblasen unmittelbar auf die gekühlte Zulaufleitung und platzen sofort. Die in der Nähe der Öffnungen um die Siedekammer bogenförmig herumgeführte Zulaufleitung wirkt somit als Schaumsperre.

Bei zylindrischer und konzentrischer Konfiguration von Siedekammer, Sammelkammer und Dampfraum ist es zweckmäßig, den Dampfraum nach oben durch einen abnehmbaren, druckdicht verschließbaren Deckel abzuschließen und unter dem Deckel einen Kräuterstutzen vorzusehen, in den der Kräutertopf eingesetzt ist. Bei abgenommenem Deckel lässt sich das Gerät bequem von oben mit frischem Wasser befüllen; das Wasser läuft dann durch den Kräuterstutzen nach unten zunächst in die Sammelkammer und sodann durch den dort vorgesehenen Überlauf und die angeschlossene Rücklaufleitung in das Vorratsgefäß. Nach dem vollständigen Befüllen wird der Kräutertopf in den Kräuterstutzen eingesetzt und anschließend der Deckel aufgesetzt. Damit ist das Gerät solange betriebsbereit, bis entweder das Vorratsgefäß vollständig leer ist oder die Kräuter im Kräutertopf ausgelaugt sind.

Ein Ausführungsbeispiel der Erfindung wird nachstehend anhand der beigefügten Zeichnungen näher erläutert. Es zeigen:
- Fig. 1a: ein Gerät zur Kräuterbedampfung mit Gehäuse, in einem Vertikalschnitt;
- Fig. 1b: das Gerät gemäß Fig. 1a, in Draufsicht;
- Fig. 2a: das Gerät ohne Gehäuse, in einem Vertikalschnitt;
- Fig. 2b: das Gerät ohne Gehäuse, in Draufsicht;
- Fig. 3a: den oberen Teil des Geräts gemäß Fig. 2a, in einem ersten Vertikalschnitt;
- Fig. 3b: den oberen Teil des Geräts gemäß Fig. 3a, in einem ersten Horizontalschnitt;
- Fig. 4a: den oberen Teil des Geräts gemäß Fig. 2a, in einem zweiten Vertikalschnitt;
- Fig. 4b: den oberen Teil des Geräts gemäß Fig. 4a, in einem zweiten Horizontalschnitt;
- Fig. 5: eine perspektivische Teilansicht des oberen Teils des Geräts von Fig. 2a.

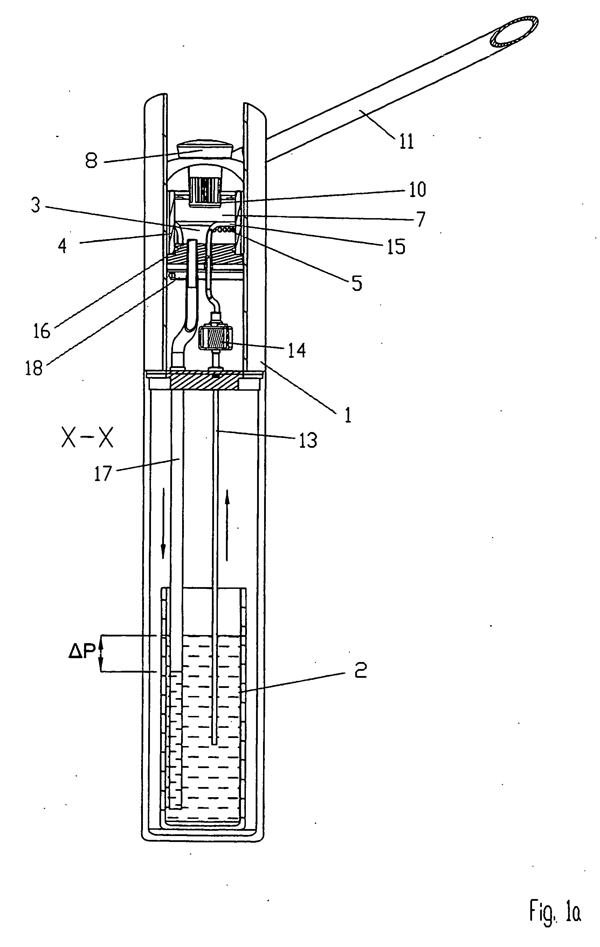

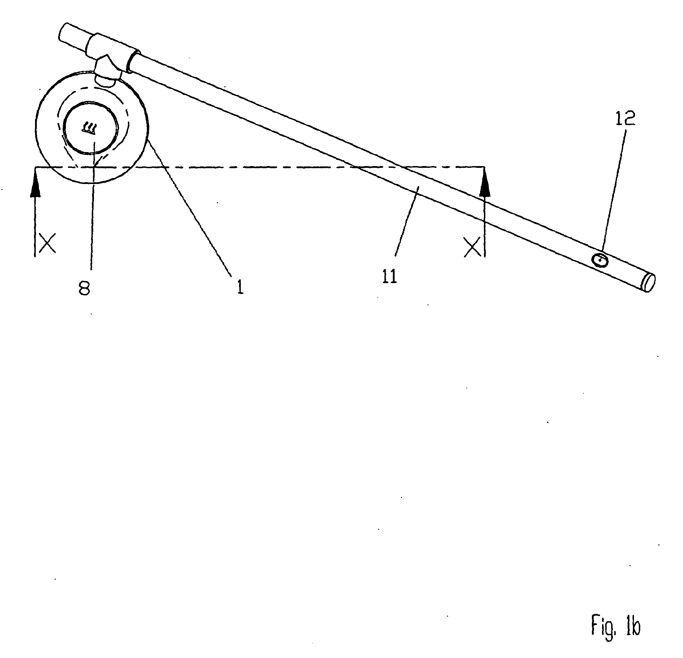

Gemäß Fig. 1a hat das erfindungsgemäße Gerät zur Kräuterbedampfung ein zylindrisches Gehäuse 1, das auf dem Boden aufsteht. Die Ebene des dargestellten Vertikalschnitts entspricht der Linie X-X in der Draufsicht von Fig. 1b.

Im unteren Teil des Gehäuses 1 ist ein Vorratsgefäß 2 für kaltes Wasser angeordnet. Im Kopfteil befindet sich eine Siedekammer 3, welche die Form eines abgestuften Zylinders hat. Die Siedekammer 3 ist nach oben hin abgeschlossen. Im oberen Abschnitt ihrer freien Seitenwand 4 ist eine Reihe nebeneinander liegender Öffnungen 5 für den Dampfaustritt vorgesehen.

Angrenzend an die Siedekammer 3 ist eine Sammelkammer 6 vorgesehen, welche den oberen freistehenden Teil der Siedekammer 3 konzentrisch umgibt. Von der Sammelkammer 6 führt ein kurzer vertikaler Kanal in den unteren Teil der Siedekammer 3.

Oberhalb der Siedekammer 3 und der Sammelkammer 6 ist ein runder Dampfraum 7 ausgebildet. Oben ist der Dampfraum 7 durch einen abnehmbaren Deckel 8 druckdicht verschlossen. Im oberen Teil des Dampfraums 7 befindet sich ein Kräutertopf 10 zur Aufnahme von pflanzlichen Kräutern. Ein Dampfrohr 11 ist seitlich an den Dampfraum 7 angeschlossen. Der in der Siedekammer 3 erzeugte und im Dampfraum 7 mit ätherischen Ölen angereicherte Dampf strömt durch eine Austrittsöffnung 12 am Ende des Dampfrohrs 11 ins Freie (vgl. Fig. 1b).

Das in dem Vorratsgefäß 2 gespeicherte Wasser gelangt durch eine Zulaufleitung 13 in die Siedekammer 3. Hierzu ist in der Zulaufleitung 13 eine elektrische Pumpe 14 vorgesehen. Der obere Teil der Zulaufleitung 13 ist in einem Bogen 15 um die Siedekammer 3 herumgeführt. Der Bogen 15 verläuft horizontal knapp oberhalb der Öffnungen 5 für den Dampfaustritt. Die Zulaufleitung 13 führt anschließend senkrecht nach unten und mündet in den unteren Teil der Siedekammer 3.

In der Sammelkammer 6 ist ein Überlauf 16 angeordnet, der in eine Rücklaufleitung 17 mündet. Die Rücklaufleitung 17 endet knapp über dem Boden des Vorratsgefäßes 2.

Unter dem Boden der Siedekammer 3 ist eine elektrische Heizplatte 18 angeordnet.

Vor der Inbetriebnahme des Geräts wird der Deckel 8 abgeschraubt und frisches Leitungswasser eingefüllt. Das Wasser sammelt sich zunächst in der Sammelkammer 6 und fließt dann durch den Überlauf 16 und die angeschlossene Rücklaufleitung 17 nach unten in das Vorratsgefäß 2. Sobald der maximale Füllstand erreicht ist, kann der Deckel 8 wieder verschlossen werden.

Nach dem Einschalten fördert die Pumpe 14 kontinuierlich kaltes Wasser aus dem Vorratsgefäß 2 durch die Zulaufleitung 13 in die Siedekammer 3. Der Wasserstand in der Siedekammer 3 und der mit dieser kommunizierenden Sammelkammer 6 wird begrenzt durch den Überlauf 16. Überschüssiges Wasser fließt selbsttätig durch die Rücklaufleitung 17 zurück in das Vorratsgefäß 2.

Ist gleichzeitig die Heizplatte 18 eingeschaltet, erhitzt sich das relativ kleine Wasservolumen in der Siedekammer 3 innerhalb von ein bis zwei Minuten bis auf Siedetemperatur. Der erzeugte Dampf tritt durch die Öffnungen 5 der Siedekammer 3 aus. Im Dampfraum 7 gelangt der Dampf dann in Kontakt mit den Kräutern bzw. Pflanzenextrakten im Kräutertopf 10. Der auf diese Weise mit ätherischen Ölen angereicherte Dampf strömt schließlich durch die Austrittsöffnung 12 des Dampfrohrs 11 ins Freie.

Der Dampfraum 7 steht über den Überlauf 16 und die Rücklaufleitung 17 mit dem Vorratsgefäß 2 in Druckverbindung. Es bildet sich ein Gleichgewicht zwischen dem Dampfdruck in der Rücklaufleitung 17 und dem auf die Wasseroberfläche im Vorratsgefäß 2 wirkenden Luftdruck und die Wassersäule. Der Druckdifferenzdruck ΔP führt dazu, dass der Wasserstand in der Rücklaufleitung 17 tiefer ist als der Füllstand im Vorratsgefäß 2. Sollte der Druck im Dampfraum 7 aufgrund einer Störung über das zulässige Maß hinaus ansteigen, so sinkt der Wasserpegel in der Rücklaufleitung 17 bis unter den Auslass. Es kann dann ein Druckausgleich zwischen Dampfraum 7 und Vorratsgefäß 2 erfolgen. Die Fluidverbindung zwischen Dampfraum 7 und Vorratsgefäß 2 über die Rücklaufleitung 17 wirkt somit als Sicherheitsventil.

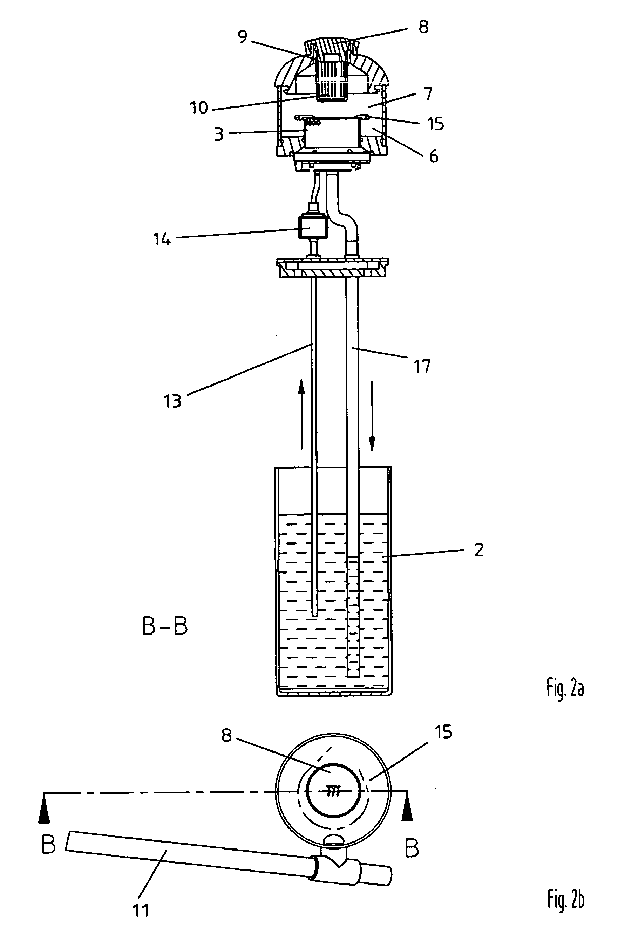

In Fig. 2a ist das Gehäuse und das Dampfrohr weggelassen. Die in Fig. 2b eingezeichnete Schnittebene B-B wurde durch den Mittelpunkt der zylindrischen Konstruktion gelegt.

In Fig. 2a ist die zylindrisch ausgebildete Siedekammer 3 gut zu erkennen. Der in der Siedekammer 3 erzeugte Dampf kann nur durch die Öffnungen 5 in der Seitenwand 4 austreten. Nach oben hin ist die Siedekammer 3 vollständig geschlossen.

Ein Kräuterstutzen 9 mit dem darin eingesetzten Kräutertopf 10 ist zentral in der Mitte über der Siedekammer 3 angeordnet. Ätherische Öle enthaltendes Kondensat kann dennoch nicht in die Siedekammer 3 hineintropfen, sondern sammelt sich in der Sammelkammer 6, welche den oberen Teil der Siedekammer 3 konzentrisch umgibt. Die Konzentration von Pflanzenextrakten bzw. ätherischen Ölen in der Siedekammer 3 ist dadurch so gering, dass sich allenfalls wenig Schaum bildet.

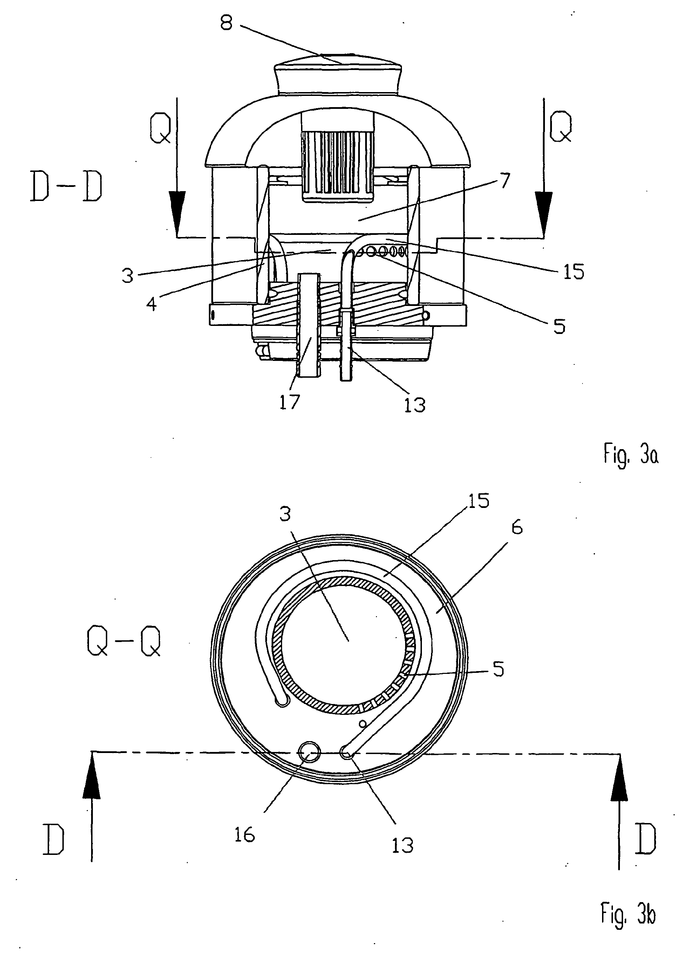

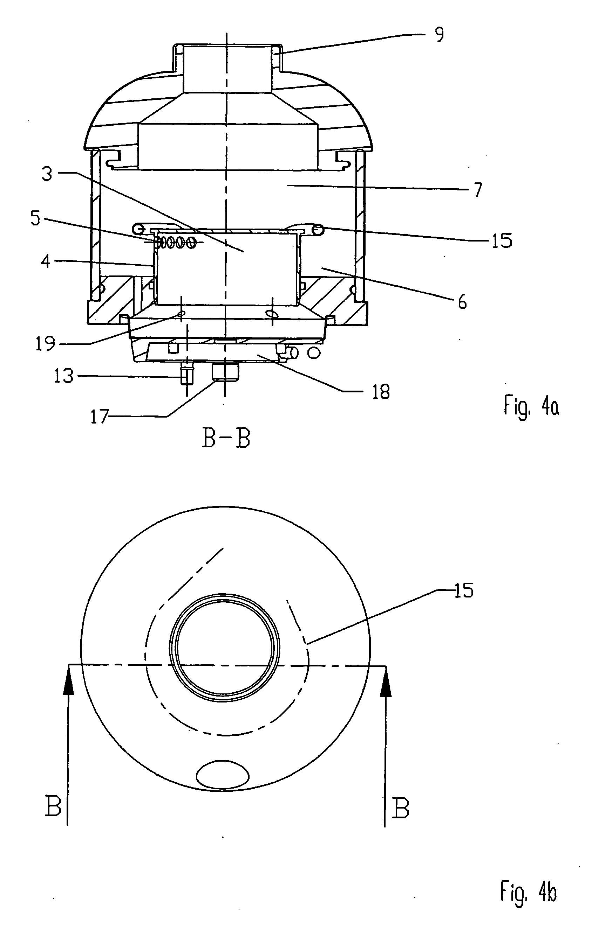

In den Detailansichten von Fig. 3a und Fig. 3b sowie Fig. 4a und Fig. 4b lässt sich besonders gut erkennen, wie die Zulaufleitung 13 in einem horizontalen Bogen 15 in geringem Abstand um die zylindrische Siedekammer 3 herumgeführt ist. Der Bogen 15 verläuft knapp oberhalb der Öffnungen 5 in der Seitenwand 4 der Siedekammer 3. Durch die Zulaufleitung 13 und den Bogen 15 fließt beständig eine kleine Menge von kaltem Wasser in die Sammelkammer 6. Der aus den Öffnungen 5 austretende heiße Dampf kommt als erstes in Kontakt mit dem Bogen 15 der Zulaufleitung 13. Hierdurch wird das zulaufende Wasser vorgewärmt. Wesentlicher ist jedoch, dass eventuell sich in der Siedekammer 3 bildender Schaum, der aus den Öffnungen 5 austritt, ebenfalls unmittelbar in Kontakt mit dem relativ kalten Bogen 15 der Zulaufleitung 13 gerät. Dadurch zerplatzen die Schaumblasen. Die Anordnung der Öffnungen 5 in der Seitenwand 4 der Siedekammer 3 in Verbindung mit der eng um die Siedekammer 3 in einem Bogen 15 herumgeführte Zulaufleitung 13 stellt eine wirksame Schaumbremse dar. Ein kleiner seitlicher Überstand des Daches der Siedekammer 3 (vgl. Fig. 4a) schützt die Öffnungen 5 zusätzlich gegen Eindringen von herabtropfendem Kondensat. Die Siedekammer 3 steht über Bohrungen 19 mit der Sammelkammer 6 in Flüssigkeitsverbindung, so dass in der Siedekammer 3 und der Sammelkammer 6 in der Regel der gleiche Flüssigkeitsstand herrscht.

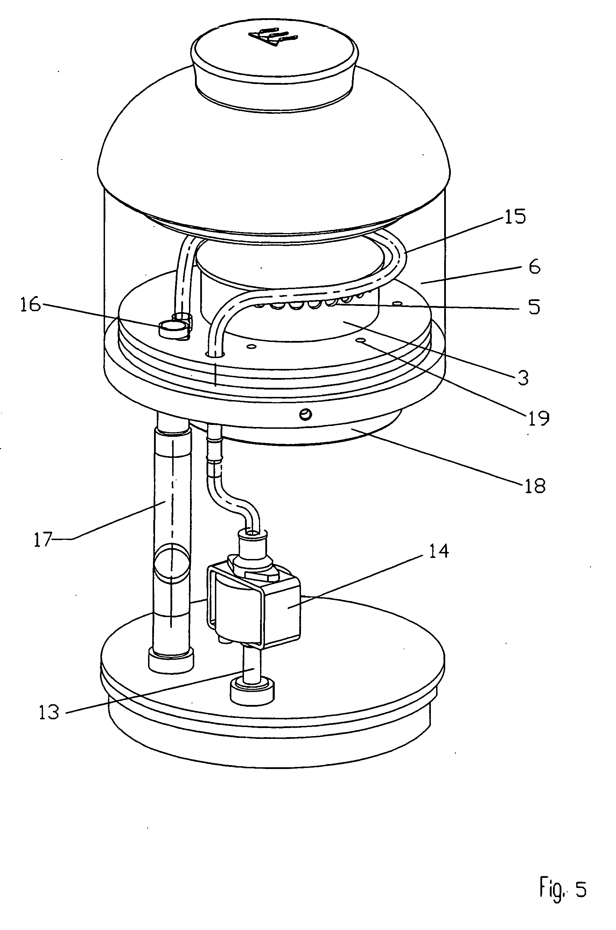

Die Perspektive von Fig. 5 zeigt insbesondere die zylindrische Siedekammer 3 mit den Öffnungen 5 für den Dampfaustritt und die Führung der Zulaufleitung 13 in einem Bogen 15 konzentrisch um den oberen Teil der Siedekammer 3 herum, bevor sie anschließend in den unteren Teil der Siedekammer 3 mündet. Die Öffnungen 5 für den Dampfaustritt befinden sich knapp unterhalb des Bogens 15. Die elektrische Pumpe 14 fördert das Wasser durch die Zulaufleitung 13. Zu erkennen ist ferner der Überlauf 16, der durch den unteren Teil der Siedekammer 3 hindurchgeführt ist und unten in die Rücklaufleitung 17 übergeht, sowie die Bohrungen 19, die eine Flüssigkeitsverbindung zwischen Siedekammer 3 und Sammelkammer 6 herstellen.

### Zusammenstellung der Bezugszeichen

- 1: Gehäuse
- 2: Vorratsgefäß
- 3: Siedekammer
- 4: Seitenwand (von 3)
- 5: Öffnungen (in 4)
- 6: Sammelkammer
- 7: Dampfraum
- 8: Deckel
- 9: Kräuterstutzen
- 10: Kräutertopf
- 11: Dampfrohr
- 12: Austrittsöffnung (von 11)
- 13: Zulaufleitung
- 14: Pumpe (in 13)
- 15: Bogen (von 13)
- 16: Überlauf
- 17: Rücklaufleitung
- 18: Heizplatte
- 19: Bohrungen (in 3)

## Patentansprüche

1. Gerät zur Kräuterbedampfung, mit
- einem Vorratsgefäß (2) für kaltes Wasser;
- einer beheizten Siedekammer (3) zur Erzeugung von Wasserdampf;
- einer Sammelkammer (6), die mit der Siedekammer (3) kommuniziert;
- einem Dampfraum (7), der mit der Siedekammer (3) in Verbindung steht;
- einer Zulaufleitung (13), über die kaltes Wasser aus dem Vorratsgefäß (2) in die Siedekammer (3) gelangt;
- einer Rücklaufleitung (17), durch die überschüssiges Wasser aus der Sammelkammer (6) zurück in das Vorratsgefäß (2) fließt;
- einem im Dampfraum (7) angeordneten Kräutertopf (10) zur Anreicherung des Dampfes mit ätherischen Ölen;
- einem an den Dampfraum (7) angeschlossenen Dampfrohr (11) mit einer Austrittsöffnung (12) für den Kräuterdampf.

2. Gerät nach Anspruch 1, **dadurch gekennzeichnet, dass** das Vorratsgefäß (12) unterhalb der Siedekammer (3) und der Sammelkammer (6) angeordnet ist.

3. Gerät nach Anspruch 2, **gekennzeichnet durch** eine Pumpe (14) in der Zulaufleitung (13).

4. Gerät nach einem der Ansprüche 1 bis 3, **gekennzeichnet durch** einen in der Sammelkammer (6) angeordneten Überlauf (16), der in die Rücklaufleitung (17) mündet und somit den Wasserstand in der Siedekammer (3) begrenzt.

5. Gerät nach einem der Ansprüche 2 bis 4, **dadurch gekennzeichnet, dass** die Rücklaufleitung (17) im Bereich des Bodens des Vorratsgefäßes (2) endet.

6. Gerät nach einem der Ansprüche 1 bis 5, **dadurch gekennzeichnet, dass**
- die Siedekammer (3) nach oben hin abgeschlossen ist und an der Seite Öffnungen (5) für den Dampfaustritt aufweist;
- die Sammelkammer (6) an die Siedekammer (3) angrenzt;
- der Dampfraum (7) oberhalb der Siedekammer (3) und der Sammelkammer (6) ausgebildet ist, so dass entstehendes Kondensat in die Sammelkammer (6) abtropft.

7. Gerät nach Anspruch 6, **dadurch gekennzeichnet, dass**
- die Siedekammer (3) zylindrisch ausgebildet ist, wobei die Öffnungen (5) für den Dampfaustritt im oberen Abschnitt der Seitenwand (6) vorgesehen sind;
- die Sammelkammer (6) zumindest den oberen Teil der Siedekammer (3) konzentrisch umgibt.

8. Gerät nach Anspruch 6 oder 7, **dadurch gekennzeichnet, dass** die Zulaufleitung (13) durch den Dampfraum (7) und an den Öffnungen (5) der Siedekammer (3) vorbei geführt ist.

9. Gerät nach Anspruch 7 und Anspruch 8, **dadurch gekennzeichnet, dass** die Zulaufleitung (13) in einem Bogen (15) um die Siedekammer (3) herum geführt ist.

10. Gerät nach einem der Ansprüche 6 bis 9, **dadurch gekennzeichnet, dass**
- der Dampfraum (7) einen abnehmbaren, druckdicht verschließbaren Deckel (8) hat;
- unter dem Deckel (8) ein Kräuterstutzen (9) vorgesehen ist, in den der Kräutertopf (10) eingesetzt ist.

## Claims

1. Device for the vaporisation of herbs, comprising:
- a storage container (2) for cold water;
- a heated boiling chamber (3) for producing steam;
- a collecting chamber (6) which communicates with the boiling chamber (3);
- a vapour chamber (7) which is connected to the boiling chamber (3);
- an inlet pipe (13) through which cold water from the storage container (2) passes into the boiling chamber (3);
- a return pipe (17) through which excess water flows from the collecting chamber (6) back into the storage container (2);
- a herb pot (10) disposed in the vapour chamber (7) for enriching the vapour with essential oils;
- a vapour pipe (11) connected to the vapour chamber (11) with an outlet opening (12) for the herbal vapour.

2. Device as claimed in Claim 1, **characterised in that** the storage container (12) is disposed below the boiling chamber (3) and the collecting chamber (6).

3. Device as claimed in Claim 2, **characterised by** a pump (14) in the inlet pipe.

4. Device as claimed in any one of Claims 1 to 3, **characterised by** an overflow (16) which is disposed in the collecting chamber (6) and opens into the return pipe and (17) and thereby limits the water level in the boiling chamber (3).

5. Device as claimed in any one of Claims 2 to 4, **characterised in that** the return pipe (17) ends in the region of the base of the storage container (2).

6. Device as claimed in any one of Claims 1 to 5, **characterised in that**
- the boiling chamber (3) is closed towards the top and has openings (5) for the vapour to escape;
- the collecting chamber (6) adjoins the boiling chamber (3);
- the vapour chamber (7) is constructed above the boiling chamber (3) and the collecting chamber (6) so that condensate which is produced drips into the collecting chamber (6).

7. Device as claimed in Claim 6, **characterised in that**
- the boiling chamber (3) is of cylindrical construction, wherein the openings (5) for the vapour to escape are provided in the upper portion of the side wall (6);
- the collecting chamber (6) concentrically surrounds at least the upper part of the boiling chamber (3).

8. Device as claimed in Claim 6 or 7, **characterised in that** the inlet pipe (13) passes through the vapour chamber (7) and past the openings (5) in the boiling chamber (3).

9. Device as claimed in Claim 7 and Claim 8, **characterised in that** the inlet pipe (13) is guided in a curve (15) around the boiling chamber (3).

10. Device as claimed in any one of Claims 6 to 9, **characterised in that**
- the vapour chamber (7) has a removable lid (8) which can be closed so as to be pressure-tight;
- a herb pipe connection (9) into which the herb pot (10) is inserted is provided below the lid (8).

## Revendications

1. Appareil d'aromathérapie comprenant
- un récipient (2) de réserve en eau froide ;
- une chambre d'ébullition chauffée (3), pour engendrer de la vapeur d'eau ;
- une chambre collectrice (6) communiquant avec la chambre d'ébullition (3) ;
- une chambre (7) à vapeur, en liaison avec la chambre d'ébullition (3) ;
- un conduit d'amenée (13), par l'intermédiaire duquel de l'eau froide parvient dans la chambre d'ébullition (3) à partir du récipient de réserve (2) ;
- Lui conduit de retour (17), par l'intermédiaire duquel de l'eau excédentaire retourne dans le récipient de réserve (2) à partir de la chambre collectrice (6) ;
- une cartouche (10) d'extraits herbacés, logée dans la chambre (7) à vapeur pour enrichir ladite vapeur en huiles essentielles ;
- un tube (11) à vapeur, raccordé à la chambre (7) à vapeur et muni d'un orifice (12) de sortie de la vapeur chargée d'extraits herbacés.

2. Appareil selon la revendication 1, **caractérisé par le fait que** le récipient de réserve (2) se trouve au-dessous de la chambre d'ébullition (3) et de la chambre collectrice (6).

3. Appareil selon la revendication 2, **caractérisé par** une pompe (14) intégrée dans le conduit d'amenée (13).

4. Appareil selon l'une des revendications 1 à 3, **caractérisé par** un trop-plein (16) disposé dans la chambre collectrice (6), qui débouche dans le conduit de retour (17) et limite ainsi le niveau d'eau dans la chambre d'ébullition (3).

5. Appareil selon l'une des revendications 2 à 4, **caractérisé par le fait que** le conduit de retour (17) s'achève dans la région du fond du récipient de réserve (2).

6. Appareil selon l'une des revendications 1 à 5, **caractérisé par le fait que**
- la chambre d'ébullition (3) est obturée vers le haut et présente, sur le côté, des orifices (5) de sortie de vapeur ;
- la chambre collectrice (6) est limitrophe de la chambre d'ébullition (3) ;
- la chambre (7) à vapeur est ménagée au-dessus de la chambre d'ébullition (3) et de la chambre collectrice (6), de sorte que du condensat généré s'écoule goutte à goutte dans ladite chambre collectrice (6).

7. Appareil selon la revendication 6, **caractérisé par le fait que**
- la chambre d'ébullition (6) est de réalisation cylindrique, les orifices (5) de sortie de vapeur étant prévus dans le tronçon supérieur de la paroi latérale (4) ;
- la chambre collectrice (6) entoure concentriquement au moins la partie supérieure de la chambre d'ébullition (3).

8. Appareil selon la revendication 6 ou 7, **caractérisé par le fait que** le conduit d'amenée (13) passe à travers la chambre (7) à vapeur et en regard des orifices (5) de la chambre d'ébullition (3).

9. Appareil selon la revendication 7 et la revendication 8, **caractérisé par le fait que** le conduit d'amenée (13) entoure la chambre d'ébullition (3) en décrivant un arc de cercle (15).

10. Appareil selon l'une des revendications 6 à 9, **caractérisé par le fait**
- **que** la chambre (7) à vapeur comporte un couvercle amovible (8), pouvant être obturé avec étanchéité à la pression ;
- **qu'**un embout (9), dans lequel la cartouche (10) d'extraits herbacés est intégrée, est prévu au-dessous du couvercle (8).
